# EUROPEAN PATENT SPECIFICATION

(11) **EP 1 663 302 B1**
(45) Date of publication and mention of the grant of the patent: **13.06.2012**
(21) Application number: 04783131.8
(22) Date of filing: 03.09.2004
(51) Int. Cl.: C12N 7/00, A61K 35/00

(54) **METHOD FOR VACCINATION OF POULTRY BY BACTERIOPHAGE LYSATE**
VERFAHREN ZUR IMPFUNG VON GEFLÜGEL MIT BAKTERIOPHAGEN-LYSAT
PROCEDE DE VACCINATION DE LA VOLAILLE PAR UN LYSAT BACTERIOPHAGE

(30) Priority: 03.09.2003 US 499339 P
(43) Date of publication of application: 07.06.2006
(73) Proprietor: Alpharma, LLC, Madison NJ 07940 (US)
(72) Inventor: PASTERNACK, Gary, R., Baltimore, MD 21210 (US); SULAKVELIDZE, Alexander, Towson, MD 21286 (US); BROWN, Torrey, Severna Park, MD 21146 (US)
(74) Representative: England, Peter Michael
(86) International application number: PCT/US2004/028776
(87) International publication number: WO 2005/027829

(56) References cited:
- EP-A2- 0 269 928
- WO-A2-01/50872
- US-A- 5 338 842
- US-B1- 6 231 871
- US-B1- 6 355 859
- GIESE M J ET AL: "THE EFFECT OF STAPHYLOCOCCUS AUREUS PHAGE LYSATE VACCINE ON A RABITMODEL OF STAPHYLOCOCCAL BLEPHARITIS, PHLYCTENULOSIS, AND CATARRHAL INFILTRATES" AMERICAN JOURNAL OF OPHTHALMOLOGY, OPHTHALMIC PUBL., CHICAGO, IL,, US, vol. 122, no. 2, 1 August 1996 (1996-08-01), pages 245-254, XP000614150 ISSN: 0002-9394
- SULAKVELIDZE ET AL: 'Mini Review, Bacteriophage Therapy' ANTIMICROBIAL AGENTS AND CHEMOTHERAPY vol. 45, no. 3, March 2001, pages 649 - 659, XP001016193

## Description

### BACKGROUND OF THE INVENTION

### 1. Field of the Invention

The present invention is directed to the field of vaccination for treatment or prevention of bacterial disease. In particular, it is directed to a phage lysate bacterin for use in vaccination of birds.

### 2. Description of Related Art

### Salmonel/a in Chickens

USDA estimates that in 50-75% of human salmonella cases the microorganism is acquired from meat, poultry, or eggs, with poultry serving as the primary vehicle of transmission. *Salmonella* are part of the normal, colonizing intestinal flora in many animals, including chickens. Studies conducted in the early 1990's by USDA indicated that 20-25% of broiler carcasses and 18% of turkey carcasses were contaminated with *Salmonella* prior to sale. See Food Safety and Inspection Service (1995); 9 CFR Part 308; Pathogen Reduction; Hazard Analysis and Critical Control Point (HACCP) Systems; Proposed Rule; 60 Fed. Reg. 6774-6889.

According to the CDC FoodNet/*Salmonella* surveillance system, the five most common human Salmonella isolates in the United States during 1990-1995 were S. *typhimurium, S. enteritidis, S. heidelberg, S. newport,* and *S. hadar.* Further, according to the USDA/FSIS data, the five most common *Salmonella* serotypes isolated from broiler chickens during the same period were *S. heidelberg, S. kentuckii, S. hadar, S. typhimurium,* and *S. thomson.*

There are strong public health, regulatory, and trade incentives for producers to reduce levels of *Salmonella* contamination in poultry. In addition to human disease, microbial contamination of animals that are very susceptible to microbial pathogens often leads to disease and increased animal morbidity. In commercial animal growing operations where animals may be crowded in facilities where other animals have been previously raised, the likelihood of such contamination is often great. This is particularly true of salmonella in the poultry industry. See Suzuki, S., "Pathogenicity of Salmonella enteriditis in poultry. International Journal of Food Microbiology 21:89-105 (1994). Infection with Salmonella species may cause significant disease in poultry flocks. Suzuki notes that non-typhoid types of Salmonella including *S. enteritidis* and *S. typhimurium* cause both overt and symptomless infections in poultry. Acute outbreaks may occur in young birds and under stress conditions with mortality rates approaching 20% in some cases and stunting of chicks of affected flocks. *S. enteritidis* may cause pericarditis, necrotic hepatic foci, induration of the yolk sack remnant, various ovarian abnormalities, peritonitis, and possible renal involvement. Consistent with this, organisms can be recovered from heart, liver, spleen, caeca, yolk sac, ovary, oviduct, peritoneum, egg, and feces of infected birds. As another example, Muir et al. ("Comparison of Salmonella typhimurium challenge models in chickens," Avian Diseases, 42:257-264, 1998) compared two different models of avian Salmonellosis using oral challenge or spread from infected litter. In both cases, *S. typhimurium* was readily recovered from both spleen and liver.

Protection from colonization by *Salmonella* requires induction of an adaptive immune response involving the mucosal immune system. The first three weeks of life are a well-known critical period during which newly hatched chicks are at risk for colonization by *Salmonella* (Smith, "The development of the flora of the alimentary tract in young animals," Journal of Pathology and Bacteriology, 89:95-122, 1965; Barnes, et al., "The intestinal flora of the chicken in the period 2 to 6 weeks of age with particular reference to the anaerobic bacteria," British Journal of Poultry Science, 13:311-326, 1972). The specific, adaptive mucosal immune response mediated by gut-associated lymphoid tissue is thought to be a critical determinant of whether or not colonization occurs (Fukotome, et al., "Intestinal mucosal immune response in chickens following intraocular immunization with liposome-associated Salmonella enterica serovar enteritidis antigen," Developmental & Comparative Immunology, 25:475-484, 2001; Muir, et al., "Immunity, vaccination and the avian intestinal tract," Developmental & Comparative Immunology, 24:325-342, 2000).

Vaccination via the avian intestinal tract produces an adaptive mucosal immune response. A number of recent studies demonstrate that oral immunization of chickens produces an adaptive immune response to the pathogen targeted by the vaccine. Allen et al., for example, using an attenuated *S. typhimurium* strain, demonstrated that oral immunization resulted in an IgA response by B cells in the intestinal lamina propria and in the spleen (Allen, et al., "Kinetics of the mucosa antibody secreting cell response and evidence of specific lymphocyte migration to the lung after oral immunization with attenuated S. enterica var. typhimurium," FEMS Immunology and Medical Microbiology, 27:275-281, 2000). Fukotome, et al., demonstrated that intraocular immunization with a liposomal *Salmonella* antigen preparation produced a secretory immune response in the intestine that was capable of inhibiting the adherence of *S. enteritidis* to HeLa cells in a model of the attachment that occurs in intestinal colonization. Immunization with *Salmonella* can protect against colonization, as shown in the studies of Van Immerseel, et al. ("The effect of vaccination with a Salmonella enteritidis aroA mutant on early cellular responses in caecal lamina propria of newly-hatched chickens," Vaccine, 20:3034-3041 2002). They showed that administration of a live mutant vaccine resulted in protection along with colonization of liver and spleen. In reviewing strategies for induction of mucosal immunity, Muir et al., described studies that used attenuated live Salmonella typhimurium as a vaccine to protect chickens against pathogenic Salmonella species (see Curtiss, et al., "Nonrecombinant and recombinant avirulent Salmonella vaccines for poultry," Veterinary Immunology and Immunopathology, 54:365-372, 1996; Hassan, et al, "Development and evaluation of an experimental vaccination program using a live avirulent Salmonella typhimurium strain to protect immunized chickens against challenge with homologous and heterologous Salmonella serotypes," Infection and Immunity, 62:5519-5527, 1994).

Immunization *in ovo* has proven very successful for agents such as Newcastle Disease virus (see Muir, et al.), but has seen little use for bacterial agents. Perhaps this is attributable to the variable immune response known to occur following oral immunization, which may, in fact, sometimes result in a suppressive response rather than a beneficial one. One instance where *in ovo* vaccination has proven to be effective is in the study of Noor and co-workers ("In ovo oral vaccination with Campylobacter jejuni establishes early development of intestinal immunity in chickens," British Journal of Poultry Science, 36:563-573, 1995), who injected heat-killed *Campylobacter* into the amniotic fluid on day 16 of incubation, followed by an oral booster in some animals on day 7 post hatch. This procedure resulted in a striking specific intestinal IgA response.

To date, vaccination approaches to reduction of *Salmonella* colonization have achieved only partial success. Approaches to *Salmonella* vaccination have employed attenuated strains (Alderton, et al., "Humoral responses and Salmonellosis protection in chickens given a vitamin-dependent Salmonella typhimurium mutant, Avian Diseases, 35:435-442, 1991; Hassan, et al., (1993) "Effect of infective dose on humoral immune responses and colonization in chickens experimentally infected with Salmonella typhimurium," Avian Diseases, 37:19-26), or preparations of Salmonella antigens prepared by sonication and detergent extraction of bacteria (Hassan, et al., (1993); Methner, et al., "Comparative study of the protective effect against Salmonella colonization in newly hatched chicks using live attenuated Salmonella vaccine strains, wild-type Salmonella strains, or a competitive exclusion product," International Journal of Food Microbiology, 35:223-230, 1993). Other immunologic approaches have employed vaccination of breeder flocks to promote maternal antibody transfer (Methner, et al., "Wirksamkeit maternaler Salmonellerantikörper gegen eine orale Testinfektion von Küken mit Salmonella enteritidis," Berliner und Münchener tierärztliche Wochenschrift, 110:373-377, 1997). It is likely that the nature of the antigen, the route of administration and anatomic localization of the adaptive immune response, and the timing of the immunization are all critical factors. Of note, there is one report in the patent literature where inoculation *in ovo* with a bacteriophage increased the hatch rate of eggs simultaneously infected with *Salmonella typhimurium* (Taylor, et al., U.S. Patent 2,851,006). This study did not, however, examine the chicks for levels of colonization.

The scientific literature describes the use of an oral *Salmonella* bacterin administered *in ovo* and post-hatch to induce an adaptive immune response and reduce the percentage of birds colonized by *Salmonella* species by acting primarily through the mucosal immune system. However, there remains a need for improved methods of protecting animals, especially poultry, against bacterial colonization and/or infection by vaccination.

### Vaccination in ovo

The desirability of injecting materials into avian eggs during incubation has been recognized for some time. Initially, the purpose of injecting eggs was to prepare various vaccines using the egg as a growth medium for the vaccine. More recent developments have involved injecting live embryonated eggs for the purpose of accomplishing some beneficial or therapeutic effect on the embryo or the bird that eventually hatches from the egg. Such beneficial effects include increased growth, decrease post-hatch mortality rates, increase the potential growth rates or eventual size of the resulting chicken, disease resistance due to *in ovo* vaccination, increased percentage hatch of incubated eggs, and otherwise improved physical characteristics of hatched poultry.

Examples of substances which have been proposed as viable treatment (or harvestable vaccine material) alternatives for delivery via in ovo injection of avian embryos include live culture vaccines, antibiotics, vitamins, and even competitive exclusion media (a live replicating organism). Specific examples of treatment substances are described in U.S. Pat. No. 4,458,630 to Sharma et al, and U.S. Pat. No. 5,028,421 to Fredericksen et al.

Several basic techniques for injecting materials into live embryonated eggs have been described, including forcing fluids through the egg shell using pressurization and physically forming an opening in the shell of an egg and then adding the desired material (e.g., injection using syringe and needle arrangements). One traditional method has been syringe injection of eggs by hand.

In order to deliver material into eggs in a routine manner, particularly in commercial egg production, it would be preferable to employ some type of automated injection devices. For example, a number of automatic devices have been disclosed for injecting eggs. These include patents to Sandhage, U.S. Pat. No. 3,377,989 and to Miller, U.S. Pat. Nos. 4,040,388; 4,469,047; and 4,593,645, and to Hebrank U.S. Pat. No. 4,681,063. Sandhage discloses a hand operated egg injection device for injecting a few eggs at the same time, but does not disclose any method or system for handling large numbers of eggs quickly and accurately. Miller '388 discloses an automated apparatus for injecting the smaller ends of eggs and resealing the holes produced, and Miller '047 shows a somewhat different automated device for injecting eggs from their larger, air sac ends. Hebrank '063 discloses an automated injection system for embryos within eggs which has an advanced fluid delivery system which eliminates the pumping of fluids through conventional fluid handling systems and thereby reduces or eliminates the possibility of the contamination of the fluid and provides a more accurate volume delivery of such fluids. There exist other alternative devices for automated injection of eggs as described more fully below.

There continues to be a pressing need to eliminate infections in poultry production both for the health benefits to the bird itself as well as to decrease the likelihood of transmitting pathogenic microorganisms through the food chain eventually to the consumer of retail chicken and turkey products. However, prior to the present invention the problems associated with all other methods of decreasing flock contamination have not been solved. Examples of such problems include the use of antibiotics in feeds and the cost of environmental decontamination of poultry production facilities.

### SUMMARY OF THE INVENTION

It is an object of this invention to provide an improved immunogenic composition for use in vaccination of birds.

This and other objectives are addressed by one or more of the following embodiments.

In one embodiment, this invention provides a phage lysate bacterin for use in vaccination of an animal in need of immunization comprising an amount of phage lysate bacterin sufficient to induce an immune response in said animal, wherein said bacterin comprises bacteriophage particles and bacterial fragments and is produced by lysis of a pathogenic bacteria by bacteriophages consisting of one or more lytic bacteriophages, and wherein said bacterin causes the animal to create antibodies or any other adaptive response against a disease caused by said pathogenic bacteria in said animal, and wherein the phage lysate bacterin is substantially free of whole, live bacteria, and wherein said animal in need of immunisation is a bird. In a preferred mode, the bacterin is administered orally. The phage lysate bacterin may be administered in a plurality of doses sequentially, and the sequential doses may be spaced over a period of one to ten weeks. In another preferred mode, at least one dose of said bacterin is administered *in ovo.*

This invention provides a phage lysate bacterin comprising bacteriophage particles and bacterial fragments produced by phage lysis, said bacterin being substantially free of whole, live bacteria.

In another embodiment, this invention provides a phage lysate bacterin produced by inoculating a suspension of live bacteria with a bacteriophage lytic for the bacteria and incubating the inoculated suspension until substantially all of the bacteria in the suspension are lysed. Alternatively, the phage lysate bacterin is produced by inoculating a suspension of live bacteria with a bacteriophage lytic for the bacteria and incubating the inoculated suspension until a substantial portion of the bacteria in the suspension are lysed, then removing substantially all whole, live bacteria from the suspension to produce the bacterin.

Hatchery eggs may be vaccinated to reduce *Salmonella* infection in a flock by (1) providing a phage lysate bacterin comprising at least one bacteriophage and (2) injecting said bacterin into a fertilized egg under conditions appropriate to decrease or eliminate colonization of the bird hatched from the egg by *Salmonella* microorganisms. Introduction of the bacterin may be carried out by injecting the bacterin *in ovo* into any compartment of the egg, including the body of the embryo. Typically, the egg into which the bacterin is introduced is incubated to hatch.

Poultry may be vaccinated to reduce bacterial colonization of a flock by (1) providing a phage lysate bacterin comprising at least one bacteriophage and (2) treating birds with the bacterin under conditions which enable the bacterin to be effective in causing a decrease or elimination of colonization of the birds by pathogenic microorganisms. The birds may be treated with the phage lysate bacterin by oral dosing in drinking water, by injection, or by spraying. The birds may be treated with bacterin at any age, such as within the first five days following hatching, on the day of hatch, or the bacterin may be delivered to the eggs by injection, including by automated injection.

### BRIEF DESCRIPTION OF THE DRAWINGS

Figure 1 is a graph comparing the reduction in *Salmonella* contamination obtained at three weeks of age by injection of eggs with bacteriophage lysate followed by spraying of chicks with the lysate.
Figure 2 is a graph comparing the reduction in *Salmonella* contamination obtained at market age by injection of eggs with bacteriophage lysate followed by spraying of chicks.
Figure 3 is a graph comparing the reduction in *Salmonella* contamination obtained at hatch by injection of eggs with bacteriophage lysate.
Figure 4 is a graph comparing the reduction in *Salmonella* contamination obtained at three weeks of age by injection of eggs with bacteriophage lysate followed by spraying of chicks, in comparison to egg injection alone or spraying of chicks alone.
Figure 5 is a graph comparing the reduction in *Salmonella* contamination obtained at market age by injection of eggs with bacteriophage lysate followed by spraying of chicks, in comparison to egg injection alone.

### DETAILED DESCRIPTION OF THE EMBODIMENTS

Bacterin is defined in medicine as a vaccine composed of weakened or dead bacteria which will cause the body to create antibodies or any other adaptive immune response against the disease normally caused by the bacteria in the vaccine.

For this invention, phage lysate bacterin is defined as a composition comprising bacteria and/or fragments of bacteria killed by lytic bacteriophage which will induce an immune response, either cellular or humoral. The bacterial components in such a composition are produced by infection of bacteria by lytic bacteriophage followed by production of new bacteriophage particles released in a subsequent lysis of the bacteria in what is termed a lytic burst. Substantially all of the bacteria in the suspension are killed by the infection, meaning that at least 90%, preferably 95%, more preferably 99% of the bacteria in the suspension are killed by the bacteriophage. Preferably, residual live bacteria are removed by means such as centrifugation or filtration so as to render the bacterin bacteriologically sterile, particularly for the bacterial host organism used to make the bacterin.

In the earliest days of bacteriophage research, it was reported that bacteriophage lysates could be effective eliciting protective immunity against a variety of bacterial strains (d'Herelle, F, Le Bacteriophage: Son Role dans l'Immunité, Paris, Masson et Cie, 1921). Many workers of the period believed that phage lysates were superior to preparations of whole bacteria for vaccination to prevent disease (Wollman, et al., "Le phénomène d'Herelle et la reaction de fixation," Comptes Rendues de la Societé de Biologie, 85:772, 1921; Hauduroy, P., Le bacteriophage de d'Hérelle, Paris, Librarie le François, 1925. pp. 161-168; Compton, A., "Immunization in experimental plague by subcutaneous inoculation with bacteriophage. (Comparison of plain and formaldehyde-treated phage-lysed plague vaccine.)," Journal of Infectious Disease, 46:152-160, 1930; Le Louet, GM., "The bacteriophage as an agent of vaccination against the 'barbone' disease," Journal of the American Veterinary Association, 67:713-717, 1925). Despite numerous examples of efficacy, phage lysates were not superior in every system examined. Perhaps due to insufficient understanding of the immune system in those days, and the variability of observed results, study of bacteriophage as an immunogen was abandoned.

More recently, Parry et al. showed that live *E. coli* were superior to heat-killed organisms at eliciting specific mucosal immunity (Parry, et al., "Intestinal immune response to E. coli antigens in the germ-free chicken," Immunology, 32:731-741, 1977). The reasons were thought to be alterations of the bacteria that led to their premature degradation in the intestinal tract. Bacteriophage-mediated lysis does not alter the bacterial cell through chemical fixation with, for example, aldehyde crosslinkers, as is done for some attenuated bacterial vaccines. Neither does it denature macromolecules, which is how heat treatment kills bacteria. Thus, phage lysates comprise a means of effectively killing bacteria while minimally altering their antigenicity. Preferably, phage lysate bacterin contains primarily bacterial fragments produced by the burst caused by lytic bacteriophage in conjunction with bacteriophage particles released by the burst.

Preparation of bacteriophage lysates which may be used in the phage lysate bacterin of this invention can be accomplished by standard procedures, such as those taught in International Publication Numbers WO01/50866, WO01/50872, WO01/51066, and 60/497,319. Typically, a culture of bacteria of the serotype for which immunity is desired is grown to an optical density at 600 nm (OD₆₀₀) of 0.1 to 0.3 in a medium suitable for culture of the bacteria, and the bacterial culture is inoculated with bacteriophage known to be lytic for the bacteria. The culture inoculated with phage is incubated under conditions favoring infection by the phage until substantially all bacteria are lysed, which typically occurs after 4 to 8 hours of fermentation, when the OD₆₀₀ can vary from 0.1 to 1.45. The resultant lysate is treated with nucleases to degrade bacterial and phage nucleic acids and filtered and/or centrifuged to remove residual whole, live bacteria prior to use as a vaccine. The phage lysate bacterin used for vaccination may contain various physiologically acceptable materials, but will preferably be substantially free of whole, live bacteria and will be sterile by culture. For oral administration, or for administration *in ovo* where the vaccine is taken up orally by the chick embryo, endotoxin values may range from 10,000 to 200,000 EU/ml as determined by *Limulus* amoebocyte lysate assay, and are most preferably greater than 60,000 EU/ml and less than or equal to 500,000 EU/ml.

Vaccination using the phage lysate bacterin is typically performed by injection or oral administration. Injection may be subcutaneously, intramuscularly, intravenously, intraperitoneally, intrapleurally, intravesicularly or intrathecally. Oral administration includes rectal, inhalation, ocular, otic, or nasal route, as well as by mouth. The phage lysate bacterin may be administered orally in, for example, mineral water, optionally with 2.0 grams of sodium bicarbonate added to reduce stomach acidity. Dose and duration of therapy will depend on a variety of factors, including the patient age, patient weight, and tolerance of the phage lysate bacterin. Typically, the vaccine may be administered in a single dose or in a series of doses spaced sequentially to enhance the immune response. The amount of bacterial and bacteriophage material in each dose may be the same or the amounts may be varied to promote the boost effect. Variations in immunization procedure within the skill of the art are within the contemplation of this invention.

The phage lysate bacterin of this invention may be used to development enhanced protective immune response in birds in need of protection from bacterial infection.

The present invention may be used in many animal husbandry industries. This includes, but is not limited to, the breeding, raising, storing, and slaughter of chickens, turkeys, ducks, geese, and other avian species. Where appropriate, the application of a bacteriophage cocktail is within the contemplation of the present invention, including a preferred mode where each bacteriophage may be grown on a separate bacterial host strain, resulting in efficacy against a broader range of bacterial strains than might be achieved by use of a single bacteriophage and host strain. In one embodiment, the working phage concentration may range from 1 x 10⁵ - 1 x 10¹⁰ pfu/ml.

The present invention provides the use of the phage lysate bacterin of the invention in the manufacture of an oral bacterin vaccine to prevent disease caused by *Salmonella* in poultry flocks by substantially reducing the percentage of birds colonized by *Salmonella* species throughout the growth cycle to market age. In a preferred embodiments, a *Salmonella* phage lysate bacterin of equal proportions of *S. enteritidis, S. agona,* and *S. newport* is prepared by fermentation and filtration. The lysate is preferably sterile with respect to bacteria. The vaccine may be administered orally by injection into hatchery eggs, for example on Day 18 of incubation, and/or sprayed onto the feathers of newly hatched chicks.

Bacterin vaccines may be produced for both typhoidal and non-typhoidal *Salmonella* species. Non-typhoidal *Salmonella* species to which bacterin vaccines are produced may include Serogroup B and D strains of *Salmonella.*

### Treatment of Fertilized Eggs

The invention contemplates the treatment of fertilized avian eggs by injection of phage lysate bacterin containing at least one bacteriophage or, more preferably, a cocktail of bacteriophage. While this embodiment describes the treatment of chickens, the invention contemplates treatment of chickens, turkeys, ducks, geese, and other avian species. After the fertilized eggs are collected in the Fertilized Egg Collection Site, the fertilized eggs may be immediately injected with phage lysate bacterin, or may be incubated and injected with phage lysate bacterin on any day up to the day of hatching. Preferably, eggs are injected between days 17 and 19 of incubation, however this may vary with the breed and the type of incubator used. Most preferably, eggs are injected on day 18 of incubation. It is common practice well known to those skilled in the art to inject fertilized eggs with vaccines to avian diseases such as Marek's disease during incubation. Phage lysate bacterin may be injected into fertilized eggs or may be mixed with vaccines and injected at any point during egg incubation. It has not been possible to consistently eliminate *Salmonella* from breeder flocks, and, consequently, *Salmonella* may be present in and on the surface of fertilized eggs; conditions in incubators promote multiplication of the organism, and chicks may become infected as they pip out of the egg. Aggressive washing of eggs and the use of disinfectants of sufficient strength to eliminate all bacterial contamination is not desirable with fertilized eggs. In this setting, injecting phage lysate bacterin directly into the eggs may provide an improved means of immunizing hatched chicks, poults, ducklings, goslings, or young of other avian species.

### Treatment of Newly Hatched Chicks and Older Birds

After the birds are hatched, the birds may be sprayed with phage lysate bacterin before they are transferred to a chicken house or to a farm. Immediately after hatching, chicks are sprayed with various viral vaccines (Newcastle, bronchitis, INDIA) which are ingested as the animals preen their feathers. A small percentage of chicks are *Salmonella*-positive at this point in time (see comments above about Salmonella on eggs); however, once introduced into chicken houses, contamination spreads rapidly to all animals in the house. Application of phage lysate bacterin immediately after hatching and before transfer to chicken houses may reduce the risk of the bacterium being spread between chicks during placement and growout. During raising in the chicken house or farm, the birds may be provided with phage lysate bacterin in their drinking water, food, or both.

### Delivery of Bacteriophage to Avian Eggs

This invention contemplates the delivery of phage lysate bacterin to eggs by injection. One traditional method of injecting eggs is injection by hand. Although skilled operators can inject eggs by hand with some success, the speed and accuracy of the process is limited. Additionally, hand injection of eggs, even by skilled operators, cannot always guarantee the continuous repeated precision delivery of materials to a desired particular location within each egg. As an alternative, active substances may be injected into eggs by automated machinery such as the Inovoject system (Embrex, Inc., Research Triangle Park, NC).

Similarly, an alternative technique for treating poultry to obtain desired results has been hand injection of very young--typically day-old--chicks. As in the hand injection of eggs, speed and precision are limited. Furthermore, injection so soon after hatch places significant stress on the young chicks.

Depending upon the purpose for which the egg is being treated, the location of injection will vary. For certain purposes, the substance to be injected into the egg needs to be delivered to the amniotic fluid near the small end of the egg, for other purposes the material needs to be delivered to the air sac end of the egg, and there may even arise occasions when a substance should be delivered to the embryo itself. Nevertheless, where eggs are being incubated to produce live poultry, care must be taken to avoid injuring the embryos during the injection and delivery of fluid substances. Individual eggs, however, can vary widely in size with accompanying associated differences in the distance between the shell and the location to which delivery of a fluid substance is desired. These differences can complicate the task of consistently supplying a desired substance to a particular location within each of a large number of eggs at a fast rate of speed.

As would be expected, when an injection device is placed in contact with a large number of eggs--which indeed is the purpose of such a machine--contamination of any one or more of the needles may occur. For example, a needle encountering an egg which has died during incubation can easily become contaminated by the materials in the dead egg.

As another consideration in egg injection, where specific vaccines or other sensitive materials are to be delivered, the specific quantity delivered is often an important parameter. This is especially true when very small quantities of materials must be delivered. For example, in some treatments of avian embryos, microliter quantities are often desired. The large systems of pumps and tubing used heretofore in injection machines makes accurate and precise delivery of such small quantities rather difficult.

Several injection devices seal the injection hole after injection to prevent leakage and contamination. U.S. Pat. No. 4,593,646 to Miller et al. discloses a method and apparatus for automatic egg injection in which support plates hold and properly position a plurality of injection devices and eggs. Each egg is sealed after injection by heat coagulating the albumin located near the injection hole. An additional sealant is then applied to the outer shell by dipping each egg into a bath of the sealant. The '646 patent does not disclose sealing the egg prior to injection.

U.S. Pat. No. 4,040,388 to Miller discloses a method and apparatus for automatic egg injection in which the downwardly facing small end of an egg is punctured. The portion of the device which punctures the egg is heated in the '388 method, allegedly sterilizing the exterior of the egg (thus preventing infection during injection) and also sealing the hole by heat coagulating a small portion of the egg albumin. The '388 patent does not disclose sealing the egg prior to injection.

U.S. Pat. No. 2,477,752 to Kiss discloses a method of injecting fertile eggs for the purpose of producing chicks having down of predetermined colors. The '752 patent discloses injecting the egg manually with a syringe and thereafter by sealing the opening in the egg. While the patent states that care should be taken to prevent air from entering the egg, no method for preventing the entrance of air is provided. Sealing the egg prior to injection is not disclosed.

U.S. Pat. No. 5,136,979 to Paul et al. discloses an apparatus and method for injecting a plurality of eggs to the same depth and location even when the eggs are of varying sizes and are misaligned. The apparatus includes a means for sterilizing the egg punch and needle sections after each injection.

U.S. Pat. No. 5,056,464 to Lewis discloses an apparatus and method for injecting a plurality of eggs in which a suction cup apparatus is used for grasping each egg.
U.S. Pat. No. 4,903,635 to Hebrank discloses a high speed automated injection system in which eggs are lifted using suction devices and separate devices are used for forming an opening in the egg shell and for injecting a fluid substance.

Some egg injection devices deliver material through the small end of an egg into the albumin. Injecting material through the large end of an egg and into the air sac above the albumin is not appropriate for delivery of all materials. Delivery into the albumin, however, increases the risk of leakage of albumin and ingress of air and contaminants after injection. Methods of injecting material into the albumin of eggs on a rapid basis should preferably provide means for preventing air and contaminants from entering the albumin, and means for preventing leakage of albumin, after injection.

Conventionally, the physical injection has been typically targeted at preferred positions within the egg in order to administer the substance into specific developing regions of the embryo. As understood by those of skill in the art, as the incubation period progresses towards maturity (i.e., hatching), the embryo and its membranes, e.g., the air cell, the allantois, and yolk sac, correspondingly change in both volume and position within the egg shell. Additionally, the quantitative volume of the enclosed fluids vary as well; for example, the density of the allantois (fluid, solid) varies as a function of time over the incubation period.

Thus, selection of both the site and time of treatment can impact the effectiveness of the injected substance as well as the mortality rate of the treated embryos. See e.g., U.S. Pat. No. 4,458,630 to Sharma et al., U.S. Pat. No. 4,681,063 to Hebrank, and U.S. Pat. No. 5,158,038 to Sheeks et al.

In one preferred embodiment, this invention contemplates the use of an automated egg injector. An example of such an injector is the Embrex Inovoject® system.

In a preferred embodiment, pathogens will be a member of the genus *Salmonella.* In a most preferable embodiment, pathogens will include *S. enteriditis, S. heidelberg, S. kentuckii, S. hadar,* and *S. typhimurium.*

### Bacteriophage Cocktails

This invention also contemplates phage lysate bacterins generated using bacteriophage cocktails, which may be custom tailored to the pathogens that are prevalent in a certain situation. Typically, pathogenic bacteria would be initially isolated from a particular source (e.g., a contaminated flock or location such as a henhouse) and susceptibility testing of the pathogens to various bacteriophage strains would be performed, analogous to antimicrobial susceptibility testing. Once each pathogen's phage susceptibility profile is determined, the appropriate phage cocktail can be formulated from phage strains to which the pathogens are susceptible and administered to the patient or applied in the environment. Since phage cocktails will typically be constructed of phage active against avian and human food-borne pathogens, isolation of such phage would be most successful from the waste of poultry farms and poultry processing plants. Typically, the phage cocktail will include one or more bacteriophage according to this invention. Thus, it may be appropriate to use certain phage cocktails in agricultural settings where there are certain human pathogens such as *Salmonella* and *Campylobacter* inherent to poultry or livestock and which contaminate the environment of such animals on an ongoing basis, resulting in a continuing source of infection by such pathogens.

Bacteriophage cocktails may be applied by contemporaneous administration of the various phage - that is, they may be applied at the same time (e.g., in the same application), or may be applied in separate applications spaced in time such that they are effective at the same time.

Other bacteria that may be lysed by phage to produce bacterin within the contemplation of the present invention include, inter alia, *Campylobacter, E. coli* O157:H7, E.coli K88 and F18 serotypes, *Acinetobacter baumanii, Actinobacillus pleuropneumoniae, Aeromonas hydrophila, Brucella abortus, Campylobacter jejuni, Clostridium perfringens, Corynebacterium bovis, Klebsiella pneumoniae, Lactococcus garvieae, Listeria* species such as *Listeria monocytogenes* and *Listeria ivanovii, Pasteurella multocida, Pasteurella haemolytica, Pseudomonas aeruginosa, Pseudomonas plecoglossicida, Shigella* species, *Staphylococcus aureus, Streptococcus equi, Vibrio anguillarum, Vibrio vulnificus, Yersinia, enterocolitica,* and *Yersinia pseudotuberculosis.* Other bacteria within the contemplation of the invention, such as for example those set forth supra, are cultured using media and methods known to those of skill in the art. These bacteria are lysed by adding bacteriophage to the culture medium following the methods described herein, and the phage concentration is optimized to target lysis which produces the maximum antigen yield. Lytic phage specific for these bacteria may be isolated by methods analogous to those described in Int. Pat. Pub. WO 01/50866.

The invention will now be described in more detail in the following nonlimiting examples with reference to the drawings. The examples are for illustration only and do not limit the scope of the present invention in any way, which is defined only by the claims. While the invention has been described with reference to specific embodiments thereof, it will be appreciated that numerous variations, modifications, and embodiments are possible, and accordingly, all such variations, modifications, and embodiments are to be regarded as being within the spirit and scope of the invention.

### EXAMPLES

### Example 1. Preparation of bacteriophage Lysate Bacterin

A phage lysate of three *Salmonella* serotypes (5 strains) is produced by independent lysis of the bacteria by six clonal bacteriophages, or monophages. *Salmonella* strains were obtained from a variety of environmental sources such as poultry production and processing facilities. Naturally occurring bacteriophages were isolated from various U.S. environmental sources, including waters of Chesapeake Bay and used without further modification. Each of the monophages was isolated using standard techniques of mixing serial dilutions of the putative phage source with top agar that was then plated over a lawn of *Salmonella* species contained in bottom agar. The plates were incubated overnight and inspected for areas of bacterial lysis, or plaques. Plaques were isolated, eluted, and re-plated serially until clonal as determined by several features including a stable restriction digest pattern, stable and homogeneous morphology by electron microscopy, and stable host range and lytic titer against host *Salmonella* strains. The particular bacteriophages are described in more detail in U.S. Provisional Patent Application No. 60/497,319. Bacteriophages are lytic tailed double-stranded DNA phages that either belong to the Siphoviridae family as classified by Ackermann and Berthiaume (1995, "Atlas of virus diagrams," CRC Press, Boca Raton, FL.), or are unclassified isometric DNA phages.

*Salmonella* phage lysate containing 3 Salmonella serotypes comprising 4 strains may be prepared as follows. A total of six monophages may be used to prepare the lysates. The *Salmonella* strains and phage used to lyse them are indicated as follows:

| **Salmonella Strains** | **Bacteriophage** |
|---|---|
| *S. Enteritidis* 378 | SPT-1 |
| *S. Enteritidis* 236 | SIT-128, SDT-15 |
| *S. Newport* 388 | SBA-178, SBA-1781 |
| *S. Agona* 121 | SSE-121 |

The isolated *Salmonella* bacteriophage SPT-1, SBA-178, SBA-1781, SIT-128, SSE-121 and SDT-15 described above were deposited on June 24, 2003 with the ATCC and received ATCC Deposit Accession Nos. PTA-5281, PTA-5284, PTA-5282, PTA-5285, PTA-5283 and PTA-5280, respectively. Separate *Salmonella* phage lysates are prepared as follows using each monophage listed in the table. These are blended in equal proportion.

Working phage stocks were concentrated clonal bacteriophage maintained in phosphate-buffered saline at 4°C in sealed glass ampoules sealed with a rubber septum and retaining ring, or as preparations prepared at the same concentration in 5% calcium gluconate and subsequently freeze-dried and stored at room temperature. Freeze-dried preparations are suspended in pyrogen-free water or sterile phosphate-buffered saline immediately before use. Individual disposable vials of bacteriophage seeds are diluted to 10⁸ pfu/ml in water, phosphate-buffered saline, or Luria broth.

Frozen bacterial seeds are rapidly thawed immediately prior to inoculation. Bacteria are prepared as an overnight culture in LB of the desired strain of *Salmonella.* Bacteria subculture is prepared by inoculating the contents of a 0.5 ml vial of the bacteria into a flask containing 70 ml of bacterial growth medium. A working seed subculture is prepared by inoculating the working seed into a flask containing an appropriate volume of bacterial growth medium. The inoculum constitutes approximately 1% of the total fermenter volume. Working seed bacterial cultures are incubated on a shaker at 150 ± 10 rpm for 1 day at 37°C ± 2°C.

Production seed culture is prepared by inoculating bacterial working seed subculture into a 10 L fermenter containing the bacterial growth medium. The inoculum constitutes approximately 5% of the total production fermenter volume. A pre-determined optimal volume of bacteriophage seed stock representing one of the monophages is added along with the inoculum. The appropriate bacteriophage seed stock(s) for the given bacterial host is added along with the inoculum. Bacteriophage are added at a predetermined multiplicity of infection (MOI) as established for each bacterial host-bacteriophage pair. Fermentation is carried out at 37°C with periodic or continuous monitoring of the OD600 until optimal lysis for each host-bacteriophage pair has occurred.

Bacterial cell suspensions containing phage are cleared of live bacteria and large bacterial fragments by either low speed centrifugation, or by tangential flow filtration. Supernatant fluids containing *Salmonella* antigens and bacteriophage are then filtered through an inert 0.22 µm pore size filter to remove intact bacteria. All filtrates are then treated with DNAse I and RNAse A. Following nuclease digestion, the *Salmonella* antigens and bacteriophage are collected, washed, concentrated, and exchanged into phosphate-buffered saline by tangential flow filtration. The tangential flow filtration process removes medium components, digested nucleic acids, and the nucleases. The resulting product is then filtered through an inert 0.22 µm filter and handled aseptically.

### Example 2. Injection of Chicken Eggs and Treatment of Chicks

The objective of this example was to determine the effects of injecting eggs and treating chicks in a commercial setting using a bacteriophage preparation specific for *Salmonella. Salmonella* species are a type of pathogenic Gram negative bacterium often found in the intestines of animals (including humans) and in the environments in which food producing animals are raised and processed (e.g., in soil, water, vegetation and on the surfaces of equipment, floors, and walls). The major pathogenic species include: *S. typhimurium, S. enteritidis, S. heidelberg, S. newport, S. hadar, S. kentuckii,* and *S. thomson. Salmonella* can cause salmonellosis, a serious and sometimes fatal illness. For a thorough discussion of *Salmonella* and the risks to human health posed by the pathogen, see US FDA CFSAN, Foodborne Pathogenic Microorganisms and Natural Toxins Handbook, Jan. 1996 with updates (available at www.cfsan.fda.gov/~mow/chap1.html), the CDC/Foodnet Salmonella surveillance system and FSIS/USDA data at http://www.foodsafety.gov/ and http://www.cdc.gov/foodnet/.

Bacteriophage employed in this experiment consisted of a cocktail of five bacteriophage directed against pathogenic *Salmonella* species. Specifically, the preparation consists of five clonal bacteriophage targeting *S. typhimurium, S. enteritidis, S. heidelberg, S. newport, S. hadar,* and *S. kentuckii.* To conduct this experiment, two egg hatchers were randomly paired. At the time of transfer of fertilized eggs to the hatcher, eggs from one hatcher were processed as usual in the commercial setting, which included injection of eggs with vaccine for Marek's disease. The eggs from the other hatcher were injected with phage specific for *Salmonella* mixed in with the Marek's vaccine. 200 ml of a bacteriophage cocktail at 10¹⁰ pfu/ml phage were introduced into a 1600 ml vaccine bag after prior removal of 200 ml of diluent to accommodate the phage. Injection of 0.1 ml of the phage/vaccine mixture per egg thus resulted in injection of a dose of 1.25 x10⁸ pfu/egg.

At the time of hatching, chicks from the control hatcher receiving Marek's vaccine but no phage were processed and moved first. 40 chicks were collected prior to spray vaccination and placed in labeled egg boxes for sampling for *Salmonella.* The remaining 960 chicks were sprayed with 7.0 ml of Newcastle/Bronchitis vaccine per 100 chicks, then taken to the farm for placement. The farm chicks remained in the chick boxes until the phage-treated chicks were delivered to the farm so that they were placed near water and food at approximately the same time.

Chicks from phage-treated eggs received a second application of phage at chick processing with spray vaccines. Again, prior to spray vaccination, 40 chicks were collected and placed in labeled egg boxes for sampling for *Salmonella.* Each box of 100 chicks was sprayed with 7.0 ml of Newcastle/Bronchitis vaccine containing bacteriophage at concentration of 2.5 x 10⁹ pfu/ml. This resulted in a dose of 1.7 x 10⁶ pfu/chick.

Chicks housed at the farm were arrayed so that no phage would be tracked into the chambers housing the control birds. Disinfectant foot pans were used and maintained at each closed chamber door. The traffic flow path was designed to isolate the phage. 960 chicks from each hatcher were placed into the trial house (1960 birds total), with 60 birds per pen.

Chicks were sampled periodically to determine the rate of contamination with *Salmonella.* For sampling of the newly hatched birds collected prior to spray vaccination, the gastrointestinal tracts were collected, diluted in buffered peptone water, held at room temperature for 48 h, then qualitatively assayed for *Salmonella* using tetrathionate enrichment and supplemental polymerase chain reaction amplification using the BAX^{®} PCR test kit (DuPont Qualicon, Inc., Wilmington, DE) according to the manufacturer's instructions.

A second group was sampled at three weeks of age. Ceca were collected from 10 birds/pen, for a total n=320. Specimen collection occurred over two days. Chick weights were recorded prior to collecting the ceca. The ceca were assayed qualitatively for the incidence of Salmonella, using tetrathionate enrichment and supplemental polymerase chain reaction amplification using the BAX^{®} PCR test kit (DuPont Qualicon, Inc., Wilmington, DE) according to the manufacturer's instructions. Any positives from the phage-treated group were isolated for phage sensitivity testing.

A third group was sampled at market age. Ceca were collected from 10 birds/pen, for a total n=160. Chick weights were recorded prior to collecting the ceca. The ceca were assayed qualitatively for the incidence of Salmonella, using tetrathionate enrichment and supplemental polymerase chain reaction amplification using the BAX^{®} PCR test kit (DuPont Qualicon, Inc., Wilmington, DE) according to the manufacturer's instructions. Any positives from the phage-treated group were isolated for phage sensitivity testing. This sampling was done at a market age of 39-40 days. Market age can vary between 42 - 60 days for broilers, depending on the target weight of the bird, and can be longer for roasters.

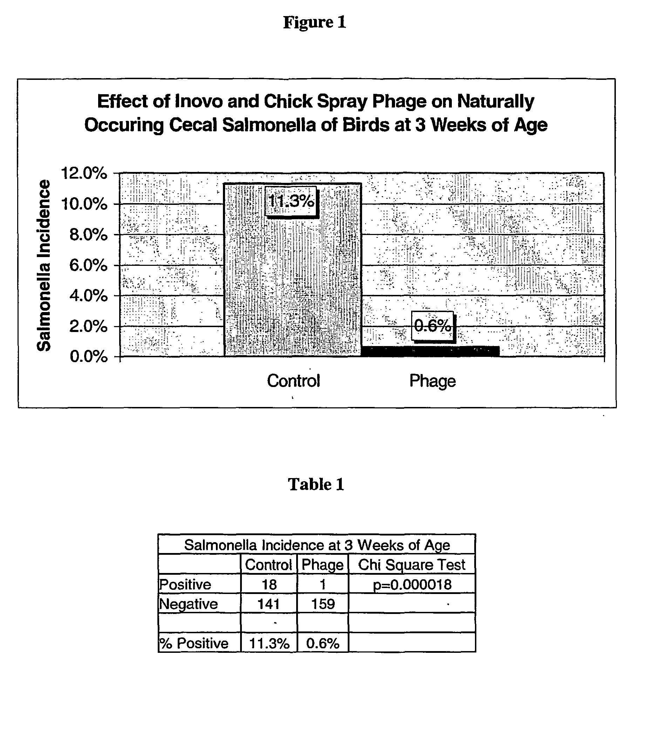

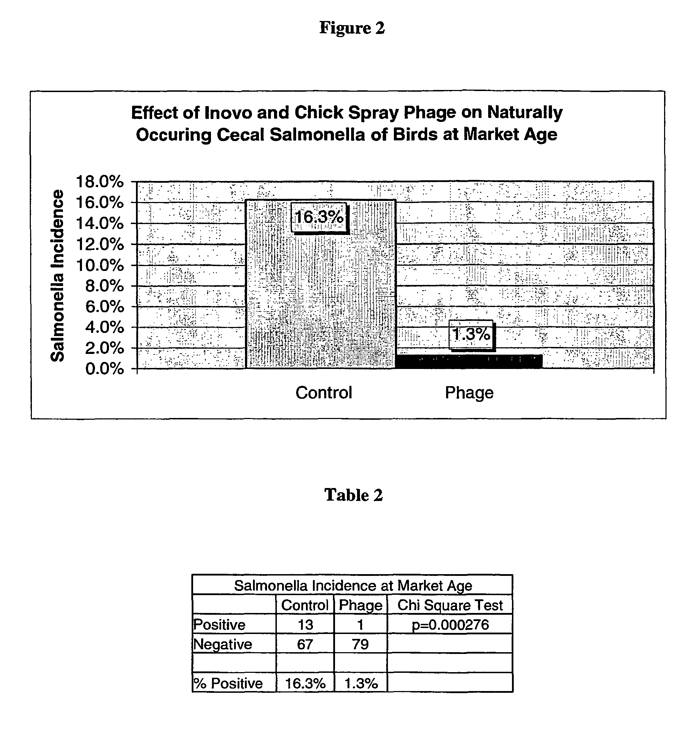

The results of this experiment are illustrated in Figures 1 & 2 with supporting data shown in Table 1 & 2. The data demonstrate that phage treatment produced a statistically significant reduction in the incidence of *Salmonella* in phage treated birds. Testing at 3 weeks of age showed that only 0.6% of phage-treated birds were *Salmonella* positive, as compared to 11 % of control birds. At market age, 1 % of phage-treated birds were *Salmonella* positive, as compared to 16%. The results indicate that application of phage in the egg combined with spray treatment of chicks is a useful intervention for the reduction of *Salmonella* during poultry production.

### Example 3. Injection of Eggs.

The objective of this example was to compare the levels of *Salmonella* reduction obtained by first intentionally contaminating eggs with *Salmonella,* then injecting eggs with a bacteriophage preparation specific for *Salmonella* to the levels obtained by spraying chicks, and to compare each group to a group treated both by injection of eggs and spraying of chicks.

Bacteriophage employed in this experiment consisted of a cocktail of five bacteriophage directed against pathogenic *Salmonella* species. Specifically, the preparation consists of five clonal bacteriophage targeting *S. typhimurium, S. enteritidis, S. heidelberg, S. newport, S. hadar, S. kentuckii. and S. thomson.* To conduct this experiment, eggs were collected from the hatchery. 40 of these eggs were randomly collected and sampled for natural *Salmonella* contamination by crushing the whole egg into 50 ml buffered peptone water and assaying for *Salmonella* as described in Example 1 above. 440 eggs were contaminated with 10⁷ cfu of a mixture of *S. Kentuckii, S. heidelburg, S. hadar,* and *S. typhimurium.* For contamination, the eggs were allowed to come to room temperature for 2 h. The *Salmonella* inoculum was prepared by combining equal aliquots from individual, overnight cultures of each strain. Each egg was inoculated with 20 µl (2 x 10 µl loops) of the *Salmonella* cocktail at room temperature. The cocktail suspension was spread over the blunt (air cell) end of the egg and allowed to dry. Following inoculation, the eggs were incubated overnight, then ten eggs were sampled as above to determine the level of *Salmonella* contamination by preparing a shell rinse sample in 50 ml buffered peptone water, an eggshell/membrane sample in 10 ml buffered peptone water, and an egg contents sample in 50 ml buffered peptone water. Each rinse and eggshell sample was serially diluted out to 10⁻⁵ or to 10⁻⁴ respectively from the initial pre-enrichment, using buffered peptone water, and all dilutions were incubated for plating. The egg contents were diluted to 10⁻² in buffered peptone water and incubated for plating. All samples and dilutions will be incubated at 37°C for 24 hours. A 10 µl aliquot of each sample was then plated and be incubated overnight. The extinction point from each dilution series of *Salmonella* will be recorded to estimate the log number of Salmonella in the original sample.

On day 18 of incubation, 50 ml of a bacteriophage cocktail at 5 x 10¹⁰ pfu/ml phage were introduced into a 400 ml Marek's vaccine bag. Injection of 0.1 ml of the phage/vaccine mixture per egg thus resulted in injection of a dose of 5.6 x 10⁸ pfu/egg. The phage preparation was identical to that described in Example 1 above. For control eggs, 50 ml of phosphate-buffered saline was introduced into an identical 400 ml Marek's vaccine bag. One group of 220 eggs was manually injected with 0.1 ml of the control preparation, while a second group of 220 eggs was injected with 0.1 ml of the phage preparation and the eggs incubated until hatching.

On the day of hatch, gastrointestinal tracts, yolk sacs, and whole bird feather rinses were collected from 20 chicks from each group. Each sample was assayed qualitatively for the presence of *Salmonella,* and 10 gastrointestinal samples and 10 feather rinse samples from each group were also quantitatively assayed by serial dilution, and plating as above.

After day-of-hatch sample collection, groups of 80 chicks were sprayed with either 28 ml of a phage solution at 5 x 10⁹ pfu/ml, or 28 ml of a control solution. At three weeks of age and at market age, ceca and feather rinses were sampled for *Salmonella* using both quantitative and qualitative assays as described above.

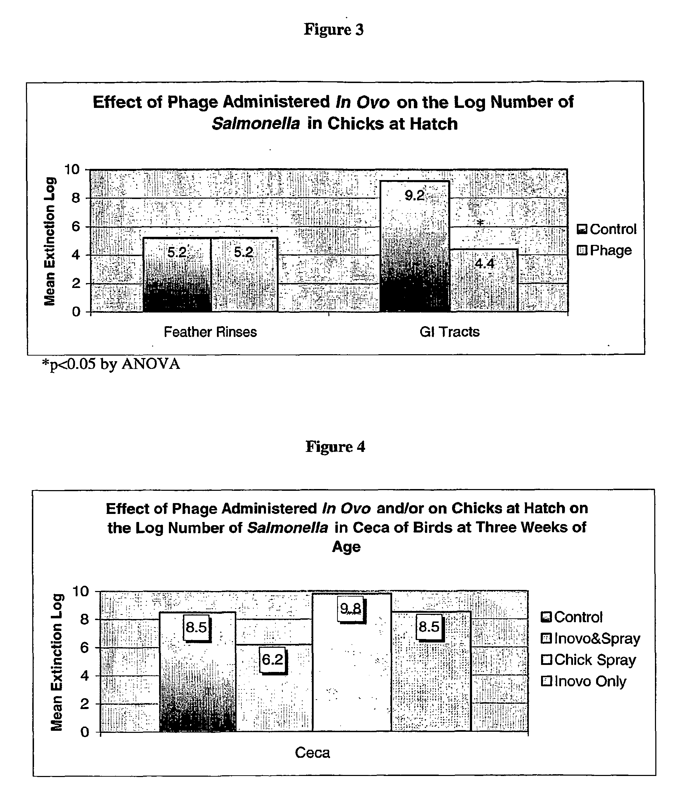

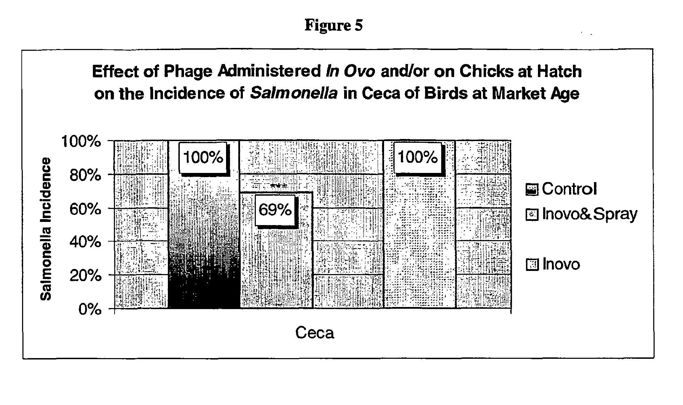

Sampling of the inoculated eggs at 24 hours documented that 100% of eggs were contaminated with *Salmonella* recoverable from whole egg washes, shell and membrane fractions, and from the egg contents. Figure 3 shows that phage treatment produces a statistically significant reduction of intentional *Salmonella* contamination in the gastrointestinal tracts but not the feathers of birds sampled at hatch. Persistent feather contamination, likely acquired during hatching from egg shells, since the shells were not previously sprayed with phage, then leads to gastrointestinal contamination of chicks from eggs injected with phage. Substantially less colonization occured in birds from injected eggs that were subsequently sprayed, as seen in Figures 4 and 5, which show the birds at 3 weeks and at market age. The principal benefit is from injection, since spraying of birds alone has little effect, as seen in Figure 4.

For purposes of charity of understanding, the foregoing invention has been described in some detail by way of illustration and example in conjunction with specific embodiments, although other aspects, advantages and modifications will be apparent to those skilled in the art to which the invention pertains.

All publications and patent applications mentioned in this specification are indicative of the level of skill of those skilled in the art to which this invention pertains.

## Claims

1. A phage lysate bacterin for use in vaccination of an animal in need of immunization comprising an amount of phage lysate bacterin sufficient to induce an immune response in said animal,
wherein said bacterin comprises bacteriophage particles and bacterial fragments and is produced by lysis of a pathogenic bacteria by bacteriophages consisting of one or more lytic bacteriophages, and
wherein said bacterin causes the animal to create antibodies or any other adaptive response against a disease caused by said pathogenic bacteria in said animal, and
wherein the phage lysate bacterin is substantially free of whole, live bacteria, and wherein said animal in need of immunisation is a bird.

2. The phage lysate bacterin of claim 1, wherein said pathogenic bacteria is *Campylobacter, E.* coli 0157: H7, *Acinetobacter baumanii, Actinobacillus pleuropneumoniae, Aeromonas hydrophila, Brucella abortus, Campylobacter jejuni, Clostridium perfringens, Corynebacterium bovis, Klebsiella pneumoniae, Lactococcus garvieae, Listeria monocytogenes, Listeria ivanovii, Pasteurella multocida, Pasteurella haemolytica, Pseudomonas aeruginosa, Pseudomonas plecoglossicida, Shigella* species, *Staphylococcus aureus, Streptococcus equi, Vibrio anguillarum,* or *Vibrio vulsificus.*

3. The phage lysate bacterin of claim 1, wherein the bacterin is administered orally.

4. The phage lysate bacterin of claim 1, wherein the phage lysate bacterin is administered in a plurality of sequential doses.

5. The phage lysate bacterin of claim 4, wherein the plurality of sequential doses is administered over a period of one to ten weeks.

6. The phage lysate bacterin of claim 1, wherein the phage lysate bacterin is administered *in ovo.*

7. Use of a phage lysate bacterin in the manufacture of a vaccine for an animal in need of immunisation comprising an amount of phage lysate bacterin sufficient to induce an immune response in said animal,
wherein the bacterin causes the animal to create antibodies or any other adaptive immune response against a disease caused by said bacteria in said animal,
wherein the bacterin comprises bacteriophage particles and bacterial fragments and is produced by lysis of a pathogenic bacteria by bacteriophages consisting of one or more lytic bacteriophages, and
wherein the bacterin is substantially free of whole, live bacteria and wherein said animal in need of immunisation is a bird.

8. The phage lysate bacterin of any one of claims 1-6, wherein said bacterin is derived from one or more pathogenic *E. coli* strains.

9. The phage bacterin of claim 8, wherein the *E. coli* strains comprise the K88 and F18 serotypes.

10. The phage bacterin of any one of claims 1-6, wherein said bacterin is derived from one or more pathogenic *Yersinia* species.

11. The phage lysate bacterin of claim 10, wherein the *Yersinia* species comprise *Yersinia pseudotuberculosis* and *Yersinia enterocolitica.*

12. The phage lysate bacterin of any one of claims 1-6, wherein said bacterin is produced by inoculating a suspension of live bacteria with a bacteriophage lytic for the bacteria and incubating the inoculated suspension until substantially all of the bacteria in the suspension are lysed.

13. The phage bacterin of any one of claims 1-6, wherein said bacterin is produced by inoculating a suspension of live bacteria with a bacteriophage lytic for the bacteria and incubating the inoculated suspension until a substantial portion of the bacteria in the suspension are lysed, then removing substantially all whole bacteria from the suspension to produce the bacterin.

14. The phage lysate bacterin of claim 1, wherein said bird is a chicken, duck, goose, or turkey.

15. The phage lysate bacterin of claim 1, wherein said bacterin provided to the bird in its drinking water, food or both.

16. The phage lysate bacterin of claim 1, wherein said bird is sprayed with bacterin.

17. The phage lysate bacterin of claim 16, wherein said bird is sprayed with bacterin immediately after hatching and before transfer to chicken houses.

18. The phage lysate bacterin of any one of claims 1-6, wherein said bacterin is derived from one or more *Salmonella* species.

19. The phage lysate bacterin of claim 18, wherein said *Salmonella* species is *S. enteriditis, S. heidelberg, S. kentuckii, S. harar, S. newport, S. agona,* or *S. typhimurium.*

20. The phage lysate bacterin of claim 1, wherein said bacterin is administered via injection.

21. The phage lysate bacterin of claim 20, wherein said bacterin is administered *in ovo* via injection.

22. The phage lysate bacterin of claim 21, wherein said bacterin is injected between days 17 and 19 of incubation.

## Patentansprüche

1. Phagen-Lysat-Bakterin zur Verwendung zur Impfung eines Tieres, das eine Immunisierung benötigt, umfassend eine Menge von Phagen-Lysat-Bakterin, die ausreicht, um eine ImmunAntwort in dem Tier auszulösen,
wobei das Bakterin Bakteriophagen-Partikel und bakterielle Fragmente umfasst und durch Lyse eines pathogenen Bakteriums durch Bakteriophagen, bestehend aus einem oder mehreren lytischen Bakteriophagen, hergestellt wird, und
wobei das Bakterin bewirkt, dass das Tier Antikörper oder irgendeine andere adaptive Antwort gegen eine Erkrankung, verursacht durch das pathogene Bakterium in dem Tier, hervorbringt, und
wobei das Phagen-Lysat-Bakterin im wesentlichen frei von ganzen, lebenden Bakterien ist, und wobei das Tier, das Immunisierung benötigt, ein Vogel ist.

2. Phagen-Lysat-Bakterin gemäß Anspruch 1, wobei das pathogene Bakterium *Campylobacter, E. coli* O157: H7, *Acinetobacter baumanii, Actinobacillus pleuropneumoniae, Aeromonas hydrophila, Brucella abortus, Campylobacter jejuni, Clostridium perfringens, Corynebacterium bovis, Klebsiella pneumoniae, Lactococcus garvieae, Listeria monocytogenes, Listeria ivanovii, Pasteurella multocida, Pasteurella haemolytica, Pseudomonas aeruginosa, Pseudomonas plecoglossicida, Shigella* Spezies, *Staphylococcus aureus, Streptococcus equi, Vibrio anguillarum* oder *Vibrio vulsificus* ist.

3. Phagen-Lysat-Bakterin gemäß Anspruch 1, wobei das Bakterin oral verabreicht wird.

4. Phagen-Lysat-Bakterin gemäß Anspruch 1, wobei das Phagen-Lysat-Bakterin in einer Vielzahl von aufeinanderfolgenden Dosen verabreicht wird.

5. Phagen-Lysat-Bakterin gemäß Anspruch 4, wobei die Vielzahl von aufeinanderfolgenden Dosen über eine Zeitspanne von 1 bis 10 Wochen verabreicht wird.

6. Phagen-Lysat-Bakterin gemäß Anspruch 1, wobei das Phagen-Lysat-Bakterin in ovo verabreicht wird.

7. Verwendung eines Phagen-Lysat-Bakterins zur Herstellung eines Impfstoffs für ein Tier, das Immunisierung benötigt, umfassend eine Menge von Phagen-Lysat-Bakterin, die ausreicht, um eine Immunantwort in dem Tier hervorzurufen,
wobei das Bakterin bewirkt, dass das Tier Antikörper oder irgendeine andere adaptive Immunantwort gegen eine Erkrankung, verursacht durch das Bakterium in dem Tier, hervorbringt,
wobei das Bakterin Bakteriophagen-Partikel und bakterielle Fragmente umfasst und durch Lyse eines pathogenen Bakteriums durch Bakteriophagen, bestehend aus einem oder mehreren lytischen Bakteriophagen, hergestellt wird, und
wobei das Bakterin im wesentlichen frei von ganzen, lebenden Bakterien ist, und wobei das Tier, das Immunisierung benötigt, ein Vogel ist.

8. Phagen-Lysat-Bakterin gemäß einem der Ansprüche 1 bis 6, wobei das Bakterin abgeleitet von einem oder von mehreren pathogenen *E. coli*-Stämmen ist.

9. Phagen-Bakterin gemäß Anspruch 8, wobei die *E. coli-*Stämme die K88- und F18-Serotypen umfassen.

10. Phagen-Bakterin gemäß einem der Ansprüche 1 bis 6, wobei das Bakterin von einer oder von mehreren pathogenen *Yersinia-*Art (en) abgeleitet ist/sind.

11. Phagen-Lysat-Bakterin gemäß Anspruch 10, wobei die *Yersinia*-Arten *Yersinia pseudotuberculosis* und *Yersinia enterocolitica* umfassen.

12. Phagen-Lysat-Bakterin gemäß einem der Ansprüche 1 bis 6, wobei das Bakterin durch Animpfen einer Suspension von lebenden Bakterien mit einem Bakteriophagen, lytisch für das Bakterium, und Inkubieren der angeimpften Suspension, bis im wesentlichen alle der Bakterien in der Suspension lysiert sind, hergestellt wird.

13. Phagen-Bakterin gemäß einem der Ansprüche 1 bis 6, wobeidas Bakterin durch Animpfen einer Suspension von lebenden Bakterien mit einem Bakteriophagen, lytisch für das Bakterium, und Inkubieren der angeimpften Suspension, bis ein wesentlicher Anteil der Bakterien in der Suspension lysiert ist, und dann Entfernen im wesentlichen aller ganzen Bakterien aus der Suspension unter Herstellung des Bakterins, hergestellt wird.

14. Phagen-Lysat-Bakterin gemäß Anspruch 1, wobei der Vogel ein Huhn, eine Ente, eine Gans oder ein Truthahn ist.

15. Phagen-Lysat-Bakterin gemäß Anspruch 1, wobei das Bakterin dem Vogel in dessen Trinkwasser, Futter oder beidem zur Verfügung gestellt wird.

16. Phagen-Lysat-Bakterin gemäß Anspruch 1, wobei der Vogel mit Bakterin besprüht wird.

17. Phagen-Lysat-Bakterin gemäß Anspruch 16, wobei der Vogel mit Bakterin unmittelbar nach dem Schlüpfen und vor dem Transfer in Hühnerställe besprüht wird.

18. Phagen-Lysat-Bakterin gemäß einem der Ansprüche 1 bis 6, wobei das Bakterin von einer oder mehreren *Salmonella*-Art(en) abgeleitet ist/sind.

19. Phagen-Lysat-Bakterin gemäß Anspruch 18, wobei die *Salmonella*-Art *S. enteriditis, S. heidelberg, S. kentuckii, S. harar, S. newport, S. agona* oder *S. typhimurium* ist.

20. Phagen-Lysat-Bakterin gemäß Anspruch 1, wobei das Bakterin mittels Injektion verabreicht wird.

21. Phagen-Lysat-Bakterin gemäß Anspruch 20, wobei das Bakterin *in ovo* mittels Injektion verabreicht wird.

22. Phagen-Lysat-Bakterin gemäß Anspruch 21, wobei das Bakterin zwischen Tag 17 und 19 der Bebrütung injiziert wird.

## Revendications

1. Bactérine de lysat de phage utilisable pour vacciner un animal ayant besoin d'être immunisé, comprenant une quantité de bactérine de lysat de phage suffisante pour induire une réponse immunitaire chez ledit animal,
dans laquelle ladite bactérine comprend des particules de bactériophages et des fragments bactériens et est produite par la lyse d'une bactérie pathogène par des bactériophages consistant en un ou plusieurs bactériophages lytiques, et
dans laquelle ladite bactérine amène l'animal à créer des anticorps ou n'importe quelle autre réponse adaptative contre une maladie provoquée par ladite bactérie pathogène chez ledit animal, et
dans laquelle la bactérine de lysat de phage est sensiblement exempte de bactéries vivantes entières et dans laquelle ledit animal ayant besoin d'être immunisé est un oiseau.

2. Bactérine de lysat de phage selon la revendication 1, dans laquelle ladite bactérie pathogène est *Campylobacter, E. coli* O157 : H7, *Acinetobacter baumanii, Actinobacillus pleuropneumoniae, Aeromonas hydrophila, Brucella abortus, Campylobacter jejuni, Clostridium perfringens, Corynebacterium bovis, Klebsiella pneumoniae, Lactococcus garvieae, Listeria monocytogenes, Listeria ivanovii, Pasteurella multocida, Pasteurella haemolytica, Pseudomonas aeruginosa, Pseudomonas plecoglossicida,* les espèces de *Shigella, Staphylococcus aureus, Streptococcus equi, vibrio anguillarum* ou *Vibrio vulsificus.*

3. Bactérine de lysat de phage selon la revendication 1, dans laquelle la bactérine est administrée par voie orale.

4. Bactérine de lysat de phage selon la revendication 1, dans laquelle la bactérine de lysat de phage est administrée dans une pluralité de doses successives.

5. Bactérine de lysat de phage selon la revendication 4, dans laquelle la pluralité de doses successives est administrée sur une période d'une à dix semaines.

6. Bactérine de lysat de phage selon la revendication 1, dans laquelle la bactérine de lysat de phage est administrée *in ovo.*

7. Utilisation d'une bactérine de lysat de phage dans la fabrication d'un vaccin destiné à un animal ayant besoin d'être immunisé, comprenant une quantité de bactérine de lysat de phage suffisante pour induire une réponse immunitaire chez ledit animal,
dans laquelle la bactérine amène l'animal à créer des anticorps ou n'importe quelle autre réponse immunitaire adaptative pour lutter contre une maladie provoquée par ladite bactérie chez ledit animal,
dans laquelle la bactérine comprend des particules de bactériophages et des fragments bactériens et est produite par la lyse d'une bactérie pathogène par des bactériophages consistant en un ou plusieurs bactériophages lytiques, et
dans laquelle la bactérine est sensiblement exempte de bactéries vivantes entières et dans laquelle ledit animal ayant besoin d'être immunisé est un oiseau.

8. Bactérine de lysat de phage selon l'une quelconque des revendications 1 à 6, dans laquelle ladite bactérine est dérivée d'une ou de plusieurs souches pathogènes d'*E. coli.*

9. Bactérine de phage selon la revendication 8, dans laquelle les souches d'*E. coli* comprennent les sérotypes K88 et F18.

10. Bactérine de phage selon l'une quelconque des revendications 1 à 6, dans laquelle ladite bactérine est dérivée d'une ou de plusieurs espèces pathogènes de *Yersinia.*

11. Bactérine de lysat de phage selon la revendication 10, dans laquelle les espèces de *Yersinia* comprennent *Yersinia pseudotuberculosis* et *Yersinia enterocolitica.*

12. Bactérine de lysat de phage selon l'une quelconque des revendications 1 à 6, dans laquelle ladite bactérine est produite par l'inoculation d'une suspension de bactéries vivantes avec un bactériophage lytique pour les bactéries et l'incubation de la suspension inoculée jusqu'à la lyse de sensiblement toutes les bactéries se trouvant dans la suspension.

13. Bactérine de phage selon l'une quelconque des revendications 1 à 6, dans laquelle ladite bactérine est produite par l'inoculation d'une suspension de bactéries vivantes avec un bactériophage lytique pour les bactéries et l'incubation de la suspension inoculée jusqu'à la lyse sensiblement d'une partie des bactéries se trouvant dans la suspension, puis l'élimination de sensiblement toutes les bactéries entières de la suspension pour produire la bactérine.

14. Bactérine de lysat de phage selon la revendication 1, dans laquelle ledit oiseau est un poulet, un canard, une oie ou une dinde.

15. Bactérine de lysat de phage selon la revendication 1, dans laquelle ladite bactérine est délivrée à l'oiseau dans son eau de consommation, sa nourriture ou les deux.

16. Bactérine de lysat de phage selon la revendication 1, dans laquelle la bactérine est pulvérisée sur ledit oiseau.

17. Bactérine de lysat de phage selon la revendication 16, dans laquelle la bactérine est pulvérisée sur ledit oiseau immédiatement après son éclosion et avant son transfert vers le poulailler.

18. Bactérine de lysat de phage selon l'une quelconque des revendications 1 à 6, dans laquelle ladite bactérine est dérivée d'une ou de plusieurs espèces de *Salmonella.*

19. Bactérine de lysat de phage selon la revendication 18, dans laquelle ladite espèce de *Salmonella* est *S. enteriditis, S. heidelberg, S. kentuckii, S. harar, S. newport, S. agona* ou *S. typhimurium.*

20. Bactérine de lysat de phage selon la revendication 1, dans laquelle ladite bactérine est administrée par injection.

21. Bactérine de lysat de phage selon la revendication 20, dans laquelle ladite bactérine est administrée *in ovo* par injection.

22. Bactérine de lysat de phage selon la revendication 21, dans laquelle ladite bactérine est injectée entre le jour 17 et le jour 19 d'incubation.
